(19) Europäisches Patentamt
European Patent Office
Office européen des brevets

(11) Veröffentlichungsnummer: **0 212 607**
**B1**

(12) **EUROPÄISCHE PATENTSCHRIFT**

(45) Veröffentlichungstag der Patentschrift: **14.03.90**

(51) Int. Cl.⁴: **C07C 317/18**, C07C 323/10

(21) Anmeldenummer: **86111387.6**

(22) Anmeldetag: **18.08.86**

(54) **Verfahren zur Herstellung von Halogenphenyl-oxethylsulfiden und deren Oxidationsprodukten.**

(30) Priorität: **28.08.85 DE 3530710**

(43) Veröffentlichungstag der Anmeldung: **04.03.87 Patentblatt 87/10**

(45) Bekanntmachung des Hinweises auf die Patenterteilung: **14.03.90 Patentblatt 90/11**

(84) Benannte Vertragsstaaten: **CH DE FR GB IT LI**

(56) Entgegenhaltungen:
**EP-A- 0 092 909**
**EP-A- 0 094 821**
**DE-A- 2 419 039**
**DE-A- 3 135 367**
**US-A- 4 297 513**

**Patent Abstracts of Japan, unexamined applications, C field, vol. 8, no. 7, 12. Januar 1984, THE PATENT OFFICE JAPANESE GOVERNEMNT Seite 42 C 204**

(73) Patentinhaber: **HOECHST AKTIENGESELLSCHAFT, Postfach 80 03 20, D-6230 Frankfurt am Main 80(DE)**

(72) Erfinder: **Papenfuhs, Theodor, Dr., Heinrich-Bleicher-Strasse 40, D- 6000 Frankfurt am Main 50(DE)**

**EP 0 212 607 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein vorteilhaftes Verfahren zur Herstellung von Halogenphenyl-oxethylsulfiden und deren Oxidationsprodukten (Halogenphenyl-oxethylsulfoxiden und Halogenphenyl-oxethylsulfonen) unter Vermeidung der den bekannten Verfahren anhaftenden Mängel.
Halogenphenyl-oxethylsulfone der allgemeinen Formel (A)

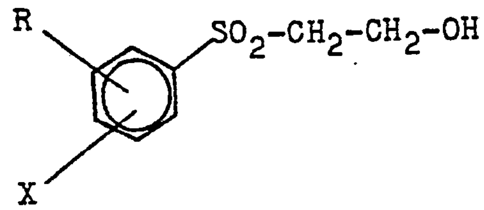

(A)

in welcher R ein Chlor- oder Bromatom, X ein Wasserstoff-, Chlor- oder Bromatom oder eine Alkyl-$C_1$-$C_6$-Gruppe bedeuten, sind wichtige Vorprodukte zur Herstellung von Reaktivfarbstoffen der Vinylsulfonreihe.
Ihre technische Herstellung erfolgte bisher aus den entsprechenden Sulfochloriden der allgemeinen Formel (B)

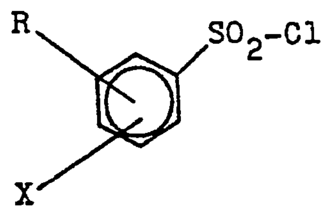

(B)

in welcher R und X die vorstehend genannten Bedeutungen haben, durch Reduktion mit Sulfit in wäßrigem Medium und anschließende Umsetzung der hierbei gebildeten Sulfinate mit Ethylenoxid (DE-OS 3 302 647 bzw. EP-OS 01 15 328), wobei die Ausgangsverbindungen der genannten Formel (B) durch Sulfochlorierung geeigneter Halogenbenzolverbindungen (DE-OS 3 302 647) oder durch Umsetzung von Benzoldiazoniumhalogeniden mit Schwefeldioxid oder Alkalihydrogensulfit in Gegenwart von Kupfer oder Kupferverbindungen (Sandmeyer-Reaktion) hergestellt werden (DE-PS 2 308 262 bzw. US-PS 3 947 512).

Dieses bekannte Verfahren ist technisch unbefriedigend, weil sowohl die erwähnten Verfahren zur Herstellung der Ausgangsverbindungen der Formel (B) als auch die sich daran anschließende Umsetzung der Sulfochloride mit erheblichen Nachteilen behaftet ist. So erfordert die Sulfochlorierung und die Sandmeyer-Reaktion einen erheblichen Aufwand zur Abwasser-Entsorgung. Die Sulfit-Reduktion ist durch einen hohen Salzanfall belastet und bei der Oxethylierung mittels Ethylenoxid werden aus Gründen der Sicherheit und Gewerbehygiene aufwendige Apparaturen benötigt.

Es bestand daher ein erheblicher Bedarf für ein anderes Verfahren zur Herstellung der Verbindungen der genannten Formel (A), durch welches die Nachteile des erwähnten bekannten technischen Verfahrens vermieden werden und eine wirtschaftlichere Herstellung der Verbindungen der Formel (A) ermöglicht wird.

Aus der Literatur (DE-OS 3 135 367; EP-OS 0 092 909) sind Verfahren bekannt geworden, Nitroaryloxethylsulfide durch Umsetzung von aktivierten Halogen-nitrobenzolen (z.B. o- bzw. p-Nitrochlorbenzol) mit Mercaptoethanol im alkalischen, ggfs. dipolar-aprotische Lösungsmittel enthaltenden Medium zugänglich zu machen, die dann durch Oxidation mit Wasserstoffperoxid in Gegenwart katalytischer Mengen Wolfram(VI)-verbindungen in die als Farbstoffvorprodukte gesuchten Nitroaryloxethylsulfone überführt werden.

Diese Verfahren gelingen jedoch nur bei den genannten stark aktivierten Halogennitrobenzolen, versagen aber beispielsweise bereits bei dem weniger aktivierten m-Nitrochlorbenzol.

Weiterhin ist aus der Literatur (EP-OS 0 094 821) ein Verfahren bekannt geworden, nichtaktivierte Halogenaromaten (algebraische Summe der δ-Hammettkonstanten zusätzlich zur Austrittsgruppe enthaltener Substituenten $\leq +0.455$), z.B. die isomeren Dichlorbenzole, mit Alkylmercaptiden Alkyl-S-Me (Me = Alkalimetall) in Gegenwart katalytisch wirkender Kronenether oder Polyglykol(-ether) bei Temperaturen von 170-190°C und Reaktionszeiten bis zu 96 Stunden zu Arylalkyl-thioethern umzusetzen und diese ggf. auch zu den entsprechenden Sulfonen zu oxidieren. Dieses Verfahren ist aus den nachstehenden Gründen ungeeignet, entsprechende nicht aktivierte Halogenaromaten der vorstehend genannten Definition (vgl. im Detail EP-OS 0 094 821, Seiten 7 bis 16) technisch mit Mercaptoethanol zu den gesuchten Halogenphenyl-oxethylsulfiden umzusetzen:

1. Es gelingt nicht, Mercaptoethanol mit Alkalimetallhydroxid umzusetzen und durch geeignetes Aufarbeiten das geforderte wasserfreie Alkalimetallsalz des Mercaptoethanols herzustellen, da dieses sich bereits unter Entwässerungsbedingungen irreversibel zersetzt.
2. Die benötigten Reaktionstemperaturen für den Halogenaustausch von >170°C bedingen eine sehr rasche Veränderung des Mercaptoethanol (-salzes), z.B. Bildung von telomeren bzw. polymeren Ethylensulfid-Derivaten bis zur Verharzung, sowie des ggf. entstehenden Zielproduktes (Wasserabspaltung zu Halogenphenylvinylsulfid).
3. Die Abtrennung der gegenüber der Zielprodukten der EP-OS 0 094 821 erheblich hydrophileren Halogenphenyloxethylsulfide von den vorgeschlagenen Katalysatoren bereitet Schwierigkeiten.

Es wurde nun überraschenderweise gefunden, daß man Verbindungen der allgemeinen Formel (1)

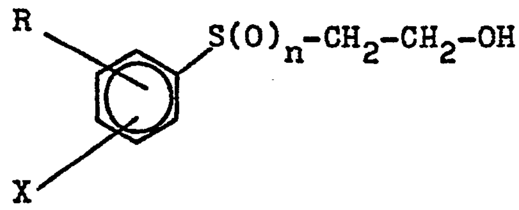

(1)

in welcher R ein Chlor- oder Bromatom, X ein Wasserstoffatom oder eine Alkyl-$C_1$-$C_6$-Gruppe bedeuten, und n die Zahl 0, 1 oder 2 darstellt, in guten Ausbeuten herstellen kann, indem man nichtaktivierte Halogenbenzole der allgemeinen Formel (2)

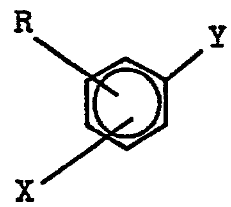

(2)

in welcher R und X die vorstehend genannten Bedeutungen haben und Y ein Chlor- oder Bromatom darstellt, mit Mercaptoethanol in einem dipolar-aprotischen Lösungsmittel in Gegenwart eines Alkalimetall-, beispielsweise Lithium-, Natrium- oder Kaliumoxids, -hydroxids oder -carbonats bei Temperaturen von 80 bis 140°C, vorzugsweise 110 bis 130°C, umsetzt zu den Halogenphenyl-oxethylsulfiden der allgemeinen Formel (3)

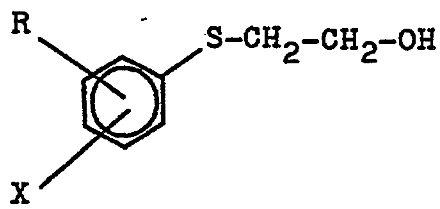

(3)

in welcher R und X die vorstehend genannten Bedeutungen haben und diese direkt in der angefallenen Reaktionsmischung oder nach Abtrennung der flüchtigen Bestandteile durch Destillation oder Extraktion und Abtrennung der festen Bestandteile durch Filtration oder Extraktion, vorzugsweise in Gegenwart von Wasser, bei einem pH-Wert von 1 bis 7, vorzugsweise von 3,5 bis 6,0, bei Temperaturen von 20°C bis 120°C, vorzugsweise von 70°C bis 100°C, mit 1 Mol Oxidationsmittel (pro Mol Halogenphenyl-oxethylsulfid) umsetzt zu den Verbindungen der Formel (1) mit n=1, oder mit mindestens 2 Mol Oxidationsmittel (pro Mol Halogenphenyl-oxethylsulfid) umsetzt zu den Verbindungen der Formel (1) mit n=2.

Erfindungsgemäß einsetzbare Ausgangsverbindungen der genannten allgemeinen Formel (2) sind beispielsweise die isomeren Dichlorbenzole und Dichlortoluole, sowie die entsprechenden Bromderivate, wobei die Dichlorbenzole, insbesondere das o- und p-Dichlorbenzol bevorzugt sind.

Als Alkalimetallverbindungen sind vor allem die Hydroxide, Oxide und Carbonate von Lithium, Natrium und Kalium zu nennen, wobei die Kaliumverbindungen insbesondere das Kaliumhydroxid, bevorzugt sind. Ihre Zugabe kann in fester Form oder in Form konzentrierter wäßriger Lösungen oder Suspensionen erfolgen.

Als dipolar-aprotische Lösungsmittel kommen N-Alkyl-$C_1$-$C_4$-pyrrolidone, Dialkyl-$C_1$-$C_4$-formamide, Tetraalkyl-$C_1$-$C_4$-harnstoffe oder Sulfolan (= Tetramethylensulfon) in Frage, wobei N-Alkyl-$C_1$-$C_4$-pyrrolidone, insbesondere N-Methylpyrrolidon, bevorzugt sind.

Von Bedeutung ist, daß die Bildung des labilen Alkalimetallsalzes des eingesetzten Mercaptoethanols erst im Zuge der Umsetzung unter Entfernen des dabei entstehenden Wassers erfolgt.

Die beim erfindungsgemäßen Verfahren anfallenden Reaktionsmischungen, welche das dipolar-aprotische Lösungsmittel, Alkalimetallhalogenid und ggf. im Überschuß eingesetztes Edukt der Formel (2) enthalten, können im Einzelfall direkt zur Oxidation eingesetzt werden. In der Regel ist jedoch eine Abtrennung der flüchtigen Bestandteile (wie dipolar-aprotisches Lösungsmittel, überschüssiges Edukt der Formel (2)) durch Destillation oder Extraktion und ggf. des Alkalihalogenids durch Filtration oder Extraktion von Vorteil.

Das dabei resultierende rohe Halogenphenyl-oxethylsulfid der Formel (1) mit n=0 kann als solches, oder nach Vakuum-destillation in reiner Form zur Oxidation eingesetzt werden, wobei je nach Menge des verwandten Oxidationsmittels und ggf. abgewandelten Oxidationsbedingungen Halogenphenyloxethylsulfoxide oder Halogenphenyl-oxethylsulfone resultieren, die von ggf. enthaltenen Begleitsubstanzen (dipolar-aprotisches Lösungsmittel, überschüssig eingesetztes Edukt der Formel (2), Alkalihalogenid, Verunreinigungen) im Zuge einer geeigneten Aufarbeitung (beispielsweise Abdestillieren flüchtiger Bestandteile, Lösen des enthaltenen Salzes, Klärfiltration von Verunreinigungen, ggf. nach Zusatz von Adsorbentien) befreit und durch Extraktion oder Filtration in reiner Form erhalten werden können.

Das Verfahren wird im einzelnen in der Weise durchgeführt, daß man eine Lösung von Mercaptoethanol in einem der genannten dipolar-aprotischen Lösungsmittel nacheinander mit der mindestens äquivalenten, in der Regel mit der einem molaren Überschuß von 5-500 %, vorzugsweise 50-200 % entsprechenden Menge Edukt der Formel (2) und mit der etwa äquivalenten Menge einer der genannten Alkaliverbindungen, wobei ein molarer Unterschuß von maximal 10 % bis zu einem molaren Überschuß von maximal 25 % möglich ist, versetzt und das Gemisch unter Rühren auf Temperaturen von 80-140°C, vorzugsweise von 110-130°C erhitzt, wobei das gebildete Wasser, ggf. zusammen mit Anteilen der in der Regel dampfflüchtigen Edukte der Formel (2) bei Normaldruck oder vorzugsweise bei einem der Reaktionstemperatur zur Erzielung eines kräftigen Rückflusses entsprechenden Vakuum abdestilliert und die ggf. mitüberdestillierte Menge an Edukt der Formel (2) auskondensiert und, vorzugsweise kontinuierlich, in den Ansatz zurückgeführt wird.

Wenn sich kein wäßriges Destillat mehr abscheidet, was in der Regel nach 4 bis 6 Stunden der Fall ist, wird noch weitere 2 bis 8 Stunden, vorzugsweise 3 bis 6 Stunden, bei Reaktionstemperatur nachgerührt, bis durch analytische Methoden, beispielsweise Gaschromatographie, kein Mercaptoethanol im Umsetzungsgemisch mehr nachweisbar ist.

Das Umsetzungsgemisch kann in Einzelfällen direkt zur Oxidation eingesetzt werden, vorteilhafter wird aber eine Aufarbeitung vorgeschaltet. Dazu wird, ggf. nach Abfiltrieren des ausgefallenen Alkalimetallhalogenids, das dipolar-aprotische Lösungsmittel im Gemisch mit nicht reagiertem Edukt der Formel (2), vorzugsweise im Vakuum, abdestilliert. Das Destillat kann dann, ergänzt um die verbrauchte Menge, im nächsten Ansatz wieder eingesetzt werden.

Der Destillations-Sumpf, bestehend aus dem Halogenphenyl-oxethylsulfid der Formel (1) mit n=0, ggf. Alkalimetallhalogenid, und möglicherweise Verunreinigungen, kann durch Vakuumfraktionierung aufgetrennt werden, wobei als Destillat in guter Ausbeute saubere Verbindungen der Formel (1) mit n=0 erhalten werden. Er kann aber auch als solcher zur Oxidation eingesetzt werden, sofern eine Abtrennung der Verunreinigungen im Zuge der Aufarbeitung des Oxidates möglich ist.

Zur Oxidation wird das Halogenphenyl-oxethylsulfid direkt in Form der angefallenen Reaktionsmischung, oder nach Abtrennung der flüchtigen und festen Bestandteile, vorzugsweise in Gegenwart von Wasser, bie einem pH-Wert von 1 bis 7, vorzugsweise von 3,5 bis 6,0 bei Temperaturen von 20 bis 120°C, vorzugsweise von 70 bis 100°C, innerhalb von 0,5 bis 5 Stunden, vorzugsweise von 1 bis 3 Stunden mit dem Oxidationsmittel versetzt und durch 1 bis 5-stündiges Nachrühren zu Ende gebracht.

Als Oxidationsmittel kommen Halogene, wie Chlor oder Brom, Hypohalogenite, wie Alkalimetallhypochlorit oder -bromit und insbesondere Wasserstoffperoxid, vorzugsweise in Gegenwart katalytischer Mengen Wolfram(VI)-verbindungen, wie Wolframtrioxid oder Alkalimetallwolframat, in Frage, wobei der geforderte pH-Wert durch Zugabe von Säuren, vorzugsweise Essigsäure, eingestellt und gehalten wird.

Die Oxidation verläuft exakt stufenweise. Bei Einsatz von 1 Mol Oxidationsmittel pro Mol Halogenphenyl-oxethylsulfid werden in guter Ausbeute die Halogenphenyl-oxethylsulfoxide der Formel (1) mit n = 1 gebildet, bei überschüssigem, mindestens 2-molarem Einsatz von Oxidationsmittel entstehen in ebenfalls guter Ausbeute die Halogenphenyl-oxethylsulfone der Formel (1) mit n = 2. Ein größerer Überschuß an Oxidationsmittel ist zwar möglich, führt aber im Falle der Halogene zu intermediärer Bildung von Halogenphenyl-β-halogenethylsulfonen, die nachfolgend erst wieder zu den Verbindungen der Formel (1) mit n = 2 gespalten werden müssen.

Zur Isolierung der Verbindungen der Formel (1) mit n = 1 oder 2 wird, ggf. nach Abdestillieren flüchtiger Bestandteile (Wasser, dipolar-aprotisches Lösungsmittel, Edukt der Formel (2)), das Halogenphenyl-oxethylsulfoxid oder -sulfon durch Filtration gewonnen, oder mit einem geeigneten Extraktionsmittel, beispielsweise Methylisobutylketon, aus dem Produktgemisch extrahiert und nach Abdampfen desselben in Form einer Schmelze isoliert.

Bei Verwendung lösungsmittelfreier Ausgangsverbindungen der Formel (1) mit n = 0 kann das in der Regel wasserlösliche Zielprodukt in seiner heißen wäßrigen Lösung, ggf. nach Zusatz eines Adsorbens,

wie beispielsweise einer Klärkohle oder Kieselerde, von unlöslichen Verunreinigungen und gelösten, adsorptiv entfernbaren Nebenprodukten geklärt und nach Abkühlen des Filtrats extraktiv oder durch Filtration isoliert werden.

Von besonderem Vorteil, und daher bevorzugt, ist die Oxidation von destillativ gereinigten Halogenphenyl-oxethylsulfiden der Formel (1) mit n = 0 mit Wasserstoffperoxid, vorzugsweise in Gegenwart katalytischer Mengen Wolfram(VI)-verbindungen, weil dann nach beendeter Reaktion die Zielprodukte direkt durch Filtration oder Phasentrennung in reiner Form isoliert werden können.

Besonders bevorzugt ist demnach eine Verfahrensweise, bei der das Umsetzungsgemisch der Halogenaustausch-Stufe destillativ in wiedereinsetzbare Edukte der Formel (2) (im Gemisch mit aprotisch dipolarem Lösungsmittel) einerseits und reines Halogenphenyl-oxethylsulfid andererseits getrennt und letzteres dann in Substanz mit der erforderlichen Menge Wasserstoffperoxid in Gegenwart katalytischer Mengen Wolfram(VI)-verbindungen bei pH 4 bis 6 zu den Zielverbindungen der Formel (1) mit n = 1 oder 2 oxidiert wird. Das neue erfindungsgemäße Verfahren vermeidet die eingangs geschilderten Nachteile bekannter Synthese-Alternativen, führt überraschenderweise in guten Ausbeuten zu reinen Verbindungen der Formel (1) (mit n = 1 oder 2) und stellt somit einen erheblichen technischen Fortschritt dar.

Die Verbindungen der Formel (1) mit n = 2 stellen, wie eingangs schon erwähnt, wichtige Vorprodukte zur Herstellung von Reaktivfarbstoffen der Vinylsulfonreihe dar; die Verbindungen der Formel (1) mit n = 0 oder 1 insofern ebenfalls, als sie durch die erfindungsgemäße Oxidation in die erstgenannten Verbindungen überführt werden können.

Beispiel 1

Eine Mischung aus 300 Teilen N-Methylpyrrolidon und 140,4 Teilen Mercaptoethanol wird unter Rühren portionsweise so mit 119,1 Teilen Ätzkali-Schuppen (88-90 %ig) versetzt, daß bei Außenkühlung mit Wasser eine Innentemperatur von 35°C nicht überschritten wird. Man rührt ca. 1 Stunde nach, wobei das Kaliumhydroxid vollständig in Lösung geht, gibt dann 300 Teile o-Dichlorbenzol zu, setzt den Ansatzkolben unter 280 Torr Vakuum und heizt auf eine Innentemperatur von 115-120°C. Dabei beginnt ein Gemisch aus Wasser und o-Dichlorbenzol abzudestillieren. Das als Oberphase in einem aufgesetzten Wasserabscheider sich sammelnde Wasser wird abgenommen, die o-Dichlorbenzol-Unterphase läuft in den Ansatz zurück. Man rührt insgesamt 15 Stunden bei 115 bis 120°C, wobei sich ca. 40 Teile Wasser abtrennen.

Nun steigert man das Vakuum auf 1-2 Torr und destilliert bei einer Sumpftemperatur von maximal 110°C o-Dichlorbenzol und N-Methylpyrrolidon im Gemisch ab. Man erhält 377,4 Teile Destillat mit einem gaschromatografisch bestimmten Gehalt von 25,8 % o-Dichlorbenzol (= 97,4 Teile) und 74,2 % N-Methylpyrrolidon (= 280,0 Teile), das beim nächsten Ansatz wieder eingesetzt werden kann.

Der verbleibende Sumpf wird nun über eine kleine Kolonne destilliert, wobei bei 1 Torr und 120 - 123°C Kopftemperatur 221,1 Teile Destillat erhalten werden, das nach Gaschromatogramm ("GC") (gegen authentischen inneren Standard) 95%ig an o-Chlorphenyl-oxethylsulfid ist (Rest: N-Methylpyrrolidon), so daß 210,0 Teile Zielprodukt der Formel

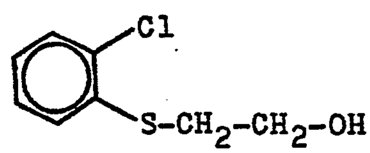

erhalten werden (Ausbeute: 80,8 % der Theorie, bezogen auf umgesetztes o-Dichlorbenzol).

Ersetzt man das N-Methylpyrrolidon durch die gleiche Menge Dimethylformamid und arbeitet sonst in der angegebenen Weise, so erhält man das o-Chlorphenyl-oxethylsulfid in vergleichbarer Reinheit, aber in ca. 5 % geringerer Ausbeute.

Beispiel 2

Zu einer Lösung von 281 Teilen Mercaptoethanol in 550 Teilen Tetramethylharnstoff werden bei einer Innentemperatur von maximal 30°C unter Rühren portionsweise 236 Teile Ätzkali-Schuppen (88-90 %ig) gegeben. Dann wird bis zur vollständigen Lösung nachgerührt. Anschließend setzt man 600 Teile p-Dichlorbenzol zu und heizt nach Evakuieren auf 350 Torr auf eine Innentemperatur von 120-125°C. Dabei beginnt ein Gemisch aus Wasser und p-Dichlorbenzol abzudestillieren, das in einem auf 55-60°C temperierten Wasserabscheider aufgefangen wird. Die wäßrige Oberphase wird abgenommen, die p-Dichlorbenzol-Unterphase läuft in den Ansatz zurück. Man rührt insgesamt 12 Stunden bei 120-125°C, wobei sich ca. 81 Teile Wasser abtrennen lassen.

Man steigert nun das Vakuum auf 4-5 Torr und destilliert bei einer Sumpftemperatur von maximal 110°C p-Dichlorbenzol und Tetramethylharnstoff im Gemisch ab. Man erhält 740 Teile Destillat, das zu

27 % aus p-Dichlorbenzol (= 200 Teile) und zu 73 % aus Tetramethylharnstoff (= 540 Teile) besteht (GC). Es kann beim nächsten Ansatz wieder eingesetzt werden. Der verbleibende Sumpf wird über eine kurze Kolonne destilliert, wobei bei 1 Torr und 132-134°C Kopftemperatur 452,7 Teile Destillat erhalten werden, das laut gaschromatografischer Analyse (gemessen gegen authentischen inneren Standard) 97,1 %ig an p-Chlorphenyl-oxethylsulfid ist (Rest: Tetramethylharnstoff), so daß 439,6 Teile Zielprodukt der Formel

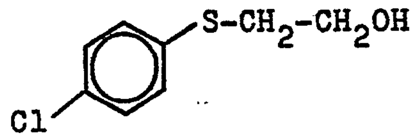

erhalten werden, was einer Ausbeute von 85,7 % der Theorie, bezogen auf umgesetztes p-Dichlorbenzol, entspricht.

Ersetzt man die Ätzkali-Schuppen durch aliquote Mengen an Ätznatron-Schuppen und arbeitet sonst in der angegebenen Weise, so wird bei ähnlicher Reinheit eine um ca. 7 % niedrigere Ausbeute erhalten.

Beispiel 3

Eine Mischung aus 300 Teilen m-Dichlorbenzol, 250 Teilen N-Methylpyrrolidon und 137,5 Teilen Mercaptoethanol wird bei 20-25°C unter Rühren und Stickstoff-Überlagerung innerhalb 30 Minuten tropfenweise mit 212 Teilen 50%iger Kalilauge versetzt. Man setzt die Apparatur unter ein Vakuum von 260-270 Torr und heizt auf 110-120°C. Innerhalb 14 Stunden lassen sich mittels aufgesetztem Wasserabscheider 145 Teile Wasser abtrennen. Das mitüberdestillierende m-Dichlorbenzol läuft kontinuierlich in den Ansatz zurück.

Bei einem Vakuum von 1-2 Torr wird nun das N-Methylpyrrolidon und nicht umgesetztes m-Dichlorbenzol im Gemisch abdestilliert (Sumpftemperatur maximal 110°C). Das Destillat (335 Teile), welches aus 230 Teilen (68,65 %) N-Methylpyrrolidon und aus 105 Teilen m-Dichlorbenzol (31,35 %) besteht, kann ohne Vorbehandlung im nächsten Ansatz wieder eingesetzt werden.

Der verbleibende Sumpf wird über eine kurze Kolonne fraktioniert. Die bei 1-2 Torr und einer Kopftemperatur von 131-134°C übergehende Fraktion (210,9 Teile) besteht zu 94,7 % aus m-Chlorphenyl-oxethylsulfid (Gaschromatogramm mit authentischem innerem Standard), der Rest ist N-Methylpyrrolidon. Demnach werden 199,5 Teile Zielprodukt der Formel

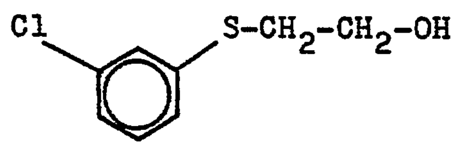

erhalten, was einer Ausbeute von 79,8 % der Theorie, bezogen auf umgesetztes m-Dichlorbenzol, entspricht.

Beispiele 4-12

Arbeitet man nach dem Verfahren des Beispiels 3, ersetzt aber das m-Dichlorbenzol durch aliquote Teile der in nachfolgender Tabelle 1 aufgeführten Ausgangsprodukte, die 50 %ige Kalilauge durch entsprechende Mengen der aus der nachstehenden Tabelle 1 ersichtlichen Alkalimetallverbindungen und das N-Methylpyrrolidon ggf. durch das dort angegebene Lösungsmittel, und arbeitet sonst in der angegebenen Weise, so erhält man die in der Tabelle 1 mit ihren Siedepunkten, Reingehalten ("RG") und Ausbeuten aufgeführten Zielverbindungen der Formel (1) mit n = 0:

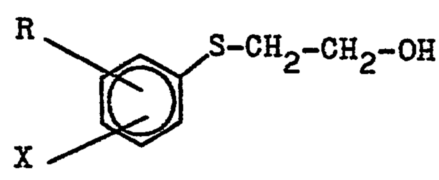

## Tabelle 1

| Beispiel | Edukt | Alkaliverbindungen | Lösungsmittel | Produkt R | Produkt X | Produkt Kp(°C)/Torr | Produkt RG (GC) | Ausbeute |
|---|---|---|---|---|---|---|---|---|
| 4 | o-Dichlorbenzol | $K_2CO_3$ | NMP | 2-Cl | H | 123/1 | 96,2 % | 60,2 % |
| 5 | o-Dichlorbenzol | Ätzkali | Sulfolan | 2-Cl | H | 123/1 | 91,4 % | 83,6 % |
| 6 | p-Dichlorbenzol | LiOH | NMP | 4-Cl | H | 134/1 | 95,7 % | 57,1 % |
| 7 | p-Dichlorbenzol | Ätznatron | NMP | 4-Cl | H | 134/1 | 95,1 % | 67,4 % |
| 8 | p-Dichlorbenzol | Ätzkali | NMP | 4-Cl | H | 134/1 | 96,2 % | 83,4 % |
| 9 | p-Dibrombenzol | Ätzkali | TMU | 4-Br | H | 141/1 | 97,4 % | 86,1 % |
| 10 | 3.4-Dichlortoluol | Ätzkali | NMP | 2-Cl | 4/5-$CH_3$ (Gemisch) | 142/1 | 92,8 % | 61,4 % |

NMP: N-Methylpyrrolidon
TMU: Tetramethylharnstoff
TBU: Tetrabutylharnstoff

Beispiel 13

Eine Emulsion aus 99 Teilen o-Chlorphenyl-oxethylsulfid (RG: 95,0 %, hergestellt nach Beispiel 1) und 400 Teilen Wasser wird unter Rühren mit Essigsäure auf pH 3 eingestellt und auf 50°C erwärmt. Man gibt nun in 15 Minuten 49 Teile 35%iges Wasserstoffperoxid zu, heizt auf 90°C und rührt 5-6 Stunden bei dieser Temperatur nach. Dann setzt man 5 Teile Aktivkohle zu, klärt nach 5 Minuten über ein beheiztes Filter und kühlt das Filtrat unter Rühren auf 10°C ab. Die dabei ausgeschiedenen farblosen Kristalle werden abgesaugt, mit Kaltwasser gewaschen und im Vakuum bei 60°C getrocknet.

Man erhält 96,3 Teile o-Chlorphenyl-oxethylsulfoxid der Formel

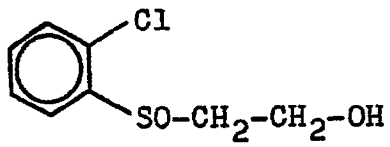

vom Schmelzpunkt 111-112°C und einem Reingehalt HPLC ("high performance liquid chromatography") von 99,1 %.

Die Ausbeute beträgt somit 93,6 % der Theorie, bezogen auf eingesetztes o-Chlorphenyl-oxethylsulfid.

Beispiel 14

Man wiederholt Beispiel 13, setzt aber statt 49 Teilen Wasserstoffperoxid 110 Teile Wasserstoffperoxid und 2,3 Teile Natriumwolframat zu. Das nach beendeter Reaktion kristallin isolierte Produkt wird im Vakuum bei 40°C getrocknet. Man erhält 108,1 Teile o-Chlorphenyl-oxethylsulfon der Formel

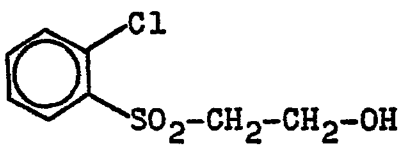

vom Schmelzpunkt 65-66°C und einem Reingehalt (HPLC) von 99,5 %. Die Ausbeute errechnet sich daraus zu 97,8 % der Theorie, bezogen auf eingesetztes o-Chlorphenyl-oxethylsulfid.

Beispiele 15 bis 18

Analog Beispiel 14 lassen sich die in der nachstehenden Tabelle 2 aufgeführten Halogenphenyl-oxethylsulfone der Formel (1) mit n = 2

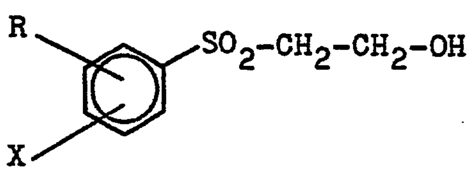

aus den entsprechenden Halogenphenyl-oxethylsulfiden (vgl. Tabelle 1) herstellen. Die erreichbaren Ausbeuten, sowie die Schmelzpunkte und Reingehalte (HPLC) sind aus der Tabelle 2 ersichtlich:

Tabelle 2

| Beispiel | R | X | Schmelzpunkt | RG (HPLC) | Ausbeute |
|---|---|---|---|---|---|
| 15 | 3-Cl | H | 40-42°C | 98,9 % | 97,5 % |
| 16 | 4-Cl | H | 52-54°C | 99,3 % | 98,4 % |
| 17 | 4-Br | H | 50-52°C | 98,5 % | 98,0 % |

Beispiel 19

Zu einer gerührten Emulsion aus 96,8 Teilen p-Chlorphenyl-oxethylsulfid (RG: 97,1 %, hergestellt nach Beispiel 2) und 250 Teilen Wasser läßt man innerhalb 60 Minuten bei Raumtemperatur 375 Teile einer 11%igen Natriumhypochlorit-Lösung tropfen. Man rührt 2 Stunden bei Raumtemperatur nach, heizt dann zum Sieden, setzt 5 Teile Aktivkohle zu und klärt nach 5 Minuten über ein beheiztes Filter. Die beim Kühlen des Filtrats auf 10°C abgeschiedenen farblosen Kristalle werden abfiltriert, mit Wasser salzfrei gewaschen und im Vakuum bei 40°C getrocknet.
Man erhält 98,2 Teile p-Chlorphenyl-oxethylsulfoxid der Formel

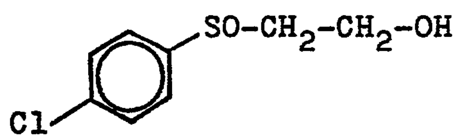

vom Schmelzpunkt 90-92°C und einem Reingehalt (HPLC) von 99,4 %. Die Ausbeute beträgt somit 95,7 % der Theorie, bezogen auf eingesetztes p-Chlorphenyl-oxethylsulfid.
Ersetzt man die Natriumhypochloritlösung durch die aliquote Menge einer 8%igen Natriumhypobromitlösung und arbeitet im übrigen wie vorstehend beschrieben, so erhält man ein vergleichbares Ergebnis.

Beispiel 20

Man verfährt, wie in Beispiel 19 beschrieben, leitet aber an Stelle der Zugabe von 375 Teilen 11%iger Natriumhypochlorit-Lösung innerhalb 2 Stunden 80 Teile Chlorgas, verdünnt mit der doppelten Menge Stickstoff, in die Emulsion ein, wobei kein Chlor aus dem Reaktionsgefäß entweichen soll.
Vor dem Hochheizen des Ansatzes zum Sieden werden zusätzlich 250 Teile Wasser zugesetzt. Die Aufarbeitung erfolgt, wie in Beispiel 19 angegeben. Man erhält 106,9 Teile p-Chlorphenyl-oxethylsulfon der Formel

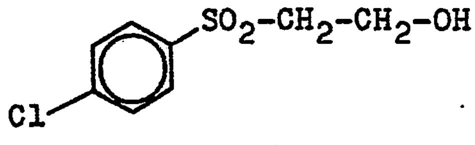

vom Schmelzpunkt 53-54°C und einem Reingehalt (HPLC) von 99,4 %. Die Ausbeute errechnet sich daraus zu 96,6 % der Theorie, bezogen auf eingesetztes p-Chlorphenyl-oxethylsulfid.

Ersetzt man das Einleiten des Chlorgases durch Zutropfen von 180 Teilen Brom und verfährt im übrigen wie beschrieben, so gelangt man zu einem vergleichbaren Ergebnis.

Beispiel 21

Der gemäß Beispiel 1 nach Abdestillieren des o-Dichlorbenzol/N-Methylpyrrolidon-Gemisches vorliegende undestillierte Sumpf wird mit 300 Teilen Wasser verrührt, durch Zugabe von ca. 50 Teilen Eisessig auf pH 5,5 eingestellt und mit 7 Teilen Natriumwolframat versetzt. Man heizt auf 55-60°C, tropft in 70 Minuten 428 Teile 35%iges Wasserstoffperoxid zu, wobei die Innentemperatur bis 95°C ansteigen darf. Man rührt 2 Stunden bei 90°C nach, kühlt auf Raumtemperatur und trennt die untere organische

Schicht ab. Man erhält 310 Teile einer Produktschmelze, die nach HPLC-Analyse (gemessen gegen authentischen inneren Standard) 80 % o-Chlorphenyl-oxethylsulfon der Formel

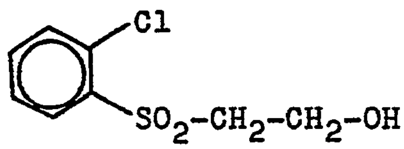

enthält, was einer Ausbeute von 248 Teilen, entsprechend 81,6 % der Theorie, bezogen auf umgesetztes o-Dichlorbenzol, entspricht.
Das Produkt kann in der vorliegenden Form direkt zu Folgereaktionen eingesetzt werden. Beispielsweise liefert es bei der Nitrierung gemäß Beispiel 5 der DE-PS 859 462 (GB-PS 735 511) reines 2-Chlor-5-nitrophenyl-oxethylsulfon vom Schmelzpunkt 166-168°C und einem Reingehalt (HPLC) von 98,5 % in 96 %iger Ausbeute.

Beispiel 22

Das gemäß Beispiel 2 nach beendeter Reaktion vorliegende, noch nicht vom p-Dichlorbenzol und Tetramethylharnstoff befreite Umsetzungsgemisch wird mit 600 Teilen Wasser vermischt. Man setzt 8 Teile Wolframtrioxid zu und stellt mit ca. 90 Teilen Eisessig einen pH-Wert von 5,5 ein.
Nun wird auf 50-55°C geheizt und innerhalb 90 Minuten durch Zulauf von 870 Teilen 35%igem Wasserstoffperoxid oxidiert, wobei die Innentemperatur auf 95°C ansteigt. Man rührt 2 Stunden bei 96°C nach, bläst das enthaltene p-Dichlorbenzol mit Wasserdampf aus (es werden 198 Teile zur Wiederverwendung beim nächsten Ansatz zurückgewonnen) und extrahiert die verbleibende wäßrige Phase erschöpfend mit Methylisobutylketon (MIBK). Der Extrakt wird im Vakuum von flüchtigen Anteilen befreit (erhalten werden 98 % des eingesetzten MIBK und 545 Teile Tetramethylharnstoff, die im Folgeansatz erneut verwendet werden können). Es verbleiben 556,5 Teile einer Produktschmelze, die nach HPLC (gemessen gegen authentischen inneren Standard) 93,8 % p-Chlorphenyl-oxethylsulfon der Formel

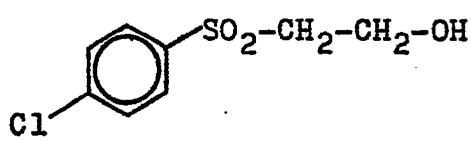

enthält, was einer Auseute von 522 Teilen, entsprechend 87 % der Theorie, bezogen auf umgesetztes p-Dichlorbenzol, entspricht.
Die Schmelze weist einen Erstarrungspunkt von 44-45°C auf und ist in dieser Form zur Durchführung von Folgereaktionen genügend rein. So liefert sie bei Nitrierung gemäß Beispiel 5 der DE-PS 859,462 in 96,4 %iger Ausbeute 4-Chlor-3-nitrophenyl-oxethylsulfon vom Schmelzpunkt 95-97°C und einem Reingehalt (HPLC) von 99,1 %.

**Patentansprüche**

1. Verfahren zur Herstellung von Verbindungen der allgemeinen Formel

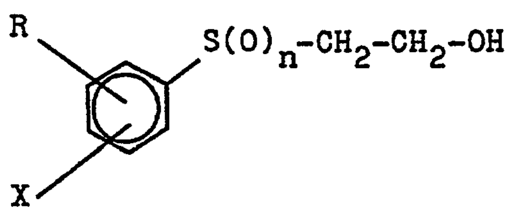

(1)

in welcher R ein Chlor- oder Bromatom, X ein Wasserstoffatom oder eine Alkyl-$C_1$-$C_6$-gruppe und n die Zahl 0, 1 oder 2 bedeuten, dadurch gekennzeichnet, daß man Halogenbenzole der allgemeinen Formel (2)

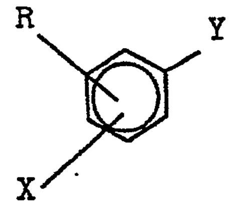

(2)

in welcher R und X die vorstehend genannten Bedeutungen haben und Y ein Chlor- oder Bromatom darstellt, mit Mercaptoethanol in einem dipolar-aprotischen Lösungsmittel in Gegenwart eines Alkalimetalloxids, -hydroxids oder -carbonats bei Temperaturen von 80 bis 140°C umsetzt zu den Halogenphenyloxethylsulfiden der allgemeinen Formel (3)

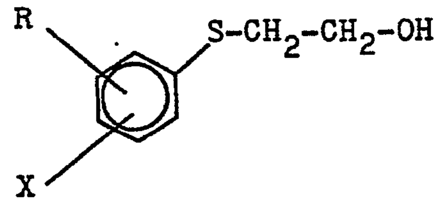

(3)

in welcher R und X die vorstehend genannten Bedeutungen haben und diese direkt in der angefallenen Reaktionsmischung oder nach Abtrennung der flüchtigen und festen Bestandteile, vorzugsweise in Gegenwart von Wasser, bei einem pH-Wert von 1 bis 7 bei Temperaturen von 20 bis 120°C mit 1 Mol Oxidationsmittel (pro Mol Halogenphenyl-oxethylsulfid) umsetzt zu den Verbindungen der Formel (1) mit n = 1, oder mit mindestens 2 Mol Oxydationsmittel (pro Mol Halogenphenyl-oxethylsulfid) umsetzt zu den Verbindungen der Formel (1) mit n = 2.

2. Verfahren nach Anspruch 1, dadurch gekennzeichnet, daß man die Umsetzung der Verbindungen der Formel (2) mit Mercaptoethanol in einem N-Alkyl-$C_1$-$C_4$-pyrrolidon, Dialkyl-$C_1$-$C_4$-formamid, Tetraalkyl-$C_1$-$C_4$-Harnstoff oder Tetramethylensulfon vornimmt.

3. Verfahren nach Anspruch 1, dadurch gekennzeichnet, daß man die Umsetzung der Verbindungen der Formel (2) mit Mercaptoethanol in N-Methylpyrrolidon, Tetramethylharnstoff, Tetrabutylharnstoff oder Dimethylformamid vornimmt.

4. Verfahren nach Anspruch 1, dadurch gekennzeichnet, daß man die Oxidation der Verbindungen der Formel (3) mit Chlor, Brom, Alkalimetallhypochlorit oder -hypobromit oder Wasserstoffperoxid vornimmt.

5. Verfahren nach Anspruch 1, dadurch gekennzeichnet, daß man die Oxidation der Verbindungen der Formel (3) mit Wasserstoffperoxid in Gegenwart von Wolframtrioxid oder Alkalimetallwolframat als Katalysatoren vornimmt.

**Claims**

1. A process for the preparation of a compound of the general formula

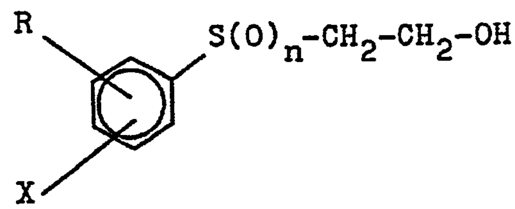

(1)

in which R denotes a chlorine or bromine atom and X denotes a hydrogen atom or a ($C_1$–$C_6$) alkyl group and n denotes the number 0, 1 or 2, wherein halo-benzenes of the general formula (2)

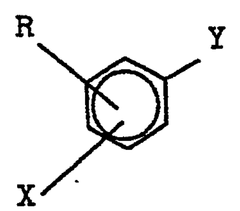

(2)

in which R and X have the meanings specified above and Y represents a chlorine or bromine atom, are reacted with mercaptoethanol in a dipolar aprotic solvent in the presence of an alkali metal oxide, hydroxide or carbonate at temperatures of 80–140°C to form halophenyl hydroxyethyl sulfides of the general formula (3)

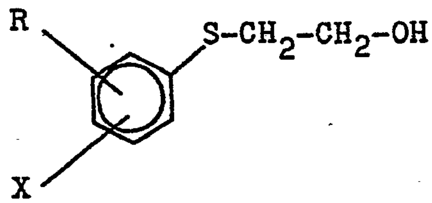

(3)

in which R and X have the meanings specified above, and these are reacted directly in the reaction mixture produced or after removal of the volatile and solid components, preferably in the presence of water, at a pH of 1 to 7 at temperatures of 20 to 120°C with 1 mol of oxidizing agent (per mol of halophenyl hydroxyethyl sulfide) to form compounds of the formula (1) with n = 1, or are reacted with at least 2 mol of oxidizing agent (per mol of halophenyl hydroxyethyl sulfide) to form the compounds of formula (1) with n = 2.

2. The process as claimed in claim 1, wherein the reaction of the compound of formula (2) with mercaptoethanol is carried out in an N-($C_1$–$C_4$)alkylpyrrolidone, di($C_1$–$C_4$)alkylformamide, tetra($C_1$–$C_4$)alkylurea or tetramethylene sulfone.

3. The process as claimed in claim 1, wherein the reaction of the compound of formula (2) with mercaptoethanol is carried out in N-methylpyrrolidone, tetramethylurea, tetrabutylurea or dimethylformamide.

4. The process as claimed in claim 1, wherein the oxidation of the compound of the formula (3) is carried out with chlorine, bromine, alkali metal hypochlorite or hypobromite or hydrogen peroxide.

5. The process as claimed in claim 1, wherein the oxidation of the compound of the formula (3) with hydrogen peroxide is carried out in the presence of tungsten trioxide or alkali metal tungstate as catalyst.

**Revendications**

1. Procédé pour la préparation de composés de formule générale

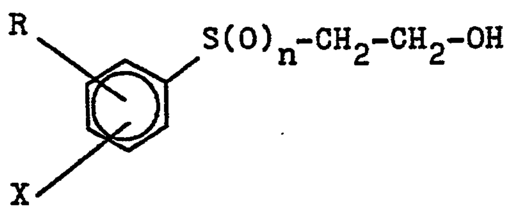

(1)

dans laquelle R est un atome de chlore ou de brome, X est un hydrogène ou un groupe alkyle en $C_1$–$C_6$ est n représente le nombre 0, 1 ou 2, caractérisé en ce qu'on fait réagir des halogénobenzènes de formule générale (2)

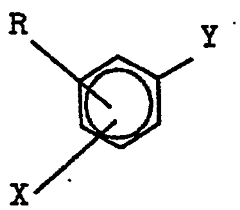

(2)

dans laquelle R et X ont les significations ci-dessus et Y représente un atome de chlore ou de brome, sur du mercaptoéthanol dans un solvant aprotique dipolaire en présence d'un oxyde, d'un hydroxyde ou d'un carbonate de métal alcalin, à des températures de 80 à 140°C, pour obtenir les sulfures d'halogénophényles et d'hydroxyéthyle de formule générale (3)

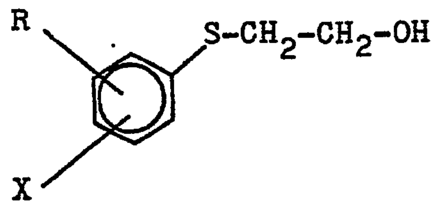

(3)

dans laquelle R et X ont les significations données ci-dessus, et qu'on les fait réagir directement dans le mélange réactionnel obtenu, ou après séparation des constituants volatils et solides, de préférence en présence d'eau, à un pH de 1 à 7 à des températures de 20 à 120°C, sur 1 mole d'un oxydant (par mole de sulfure d'halogénophényle et d'hydroxyéthyle), pour obtenir les composés de formule (1) avec n = 1, ou encore qu'on les fait réagir avec au moins 2 moles d'un oxydant (par mole de sulfures d'halogénophényles et d'hydroxyéthyle), pour donner les composés de formule (1) avec n = 2.

2. Procédé selon la revendication 1, caractérisé en ce qu'on met en œuvre la réaction des composés de formule (2) sur le mercaptoéthanol dans une N-(alkyle en $C_1$–$C_4$)-pyrrolidone, un di(alkyle $C_1$–$C_4$)-formamide, une tétra-(alkyle en $C_1$–$C_4$)-urée ou de la tétraméthylènesulfone.

3. Procédé selon la revendication 1, caractérisé en ce qu'on met en œuvre la réaction des composés de formule (2) sur le mercaptoéthanol dans de la tétrabutylurée ou du diméthylformamide.

4. Procédé selon la revendication 1, caractérisé en ce qu'on met en œuvre l'oxydation des composés de formule (3) avec du chlore, du brome, un hypochlorite ou un hypobromite de métal alcalin, ou du peroxyde d'hydrogène.

5. Procédé selon la revendication 1, caractérisé en ce qu'on met en œuvre l'oxydation des composés de formule (3) avec du peroxyde d'hydrogène en présence de trioxyde de tungstène ou d'un tungstate de métal alcalin servant de catalyseurs.